**Europäisches Patentamt**

**European Patent Office**

**Office européen des brevets**

(19)

(11) Numéro de publication: **0 192 575**
**B1**

# (12) FASCICULE DE BREVET EUROPEEN

(45) Date de publication du fascicule du brevet:
**26.04.89**

(21) Numéro de dépôt: **86400346.2**

(22) Date de dépôt: **19.02.86**

(51) Int. Cl.⁴: **A61M 1/10**

(54) **Pompe de perfusion coronaire.**

(30) Priorité: **20.02.85 FR 8502429**

(43) Date de publication de la demande:
**27.08.86 Bulletin 86/35**

(45) Mention de la délivrance du brevet:
**26.04.89 Bulletin 89/17**

(84) Etats contractants désignés:
**AT BE CH DE FR GB IT LI LU NL SE**

(56) Documents cités:
**FR-A- 2 361 123**
**FR-A- 2 502 499**
**US-A- 3 585 983**
**US-A- 4 284 073**
**US-A- 4 391 276**

(73) Titulaire: **MEDICORP RESEARCH LABORATORIES CORPORATION, 1200 North Federal Highway Suite 200-26, Boca Raton Florida 33432(US)**

(72) Inventeur: **Karcher, Gilles, 2, Rue Lafayette, F-54000 Nancy(FR)**
Inventeur: **Amor, Max, 9, Square de Liège, F-54500 Vandoeuvre(FR)**
Inventeur: **Niddam, Roger, 43, Allée du Jardin Anglais, F-93340 Le Rancy(FR)**
Inventeur: **Villemot, Jean-Pierre, Le Trident Rue Cyffle, F-54000 Nancy(FR)**

(74) Mandataire: **Laget, Jean-Loup et al, Cabinet Pierre Loyer 77, rue Boissière, F-75116 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne une pompe de perfusion coronaire, susceptible d'apporter une assistance thérapeutique en cas d'insuffisance de la circulation coronaire.

Cette insuffisance est une situation fréquemment rencontrée, qui est actuellement traitée médicalement ou chirurgicalement. Ces traitements nécessitent un appoint thérapeutique de quelques heures ou de quelques jours, cap au-delà duquel le malade peut retrouver une autonomie secondaire.

Le problème posé par le rétrécissement d'une artère coronaire n'est pas simple à résoudre. En effet, en aval du rétrécissement, on assiste à l'apparition d'un état ischémique dû à une réaction de protection des cellules. Pour rétablir la circulation sanguine, on peut supprimer le rétrécissement au moyen d'une intervention chirurgicale, telle qu'une dilatation de l'artère par un ballonnet gonflable, par exemple. Mais si l'on rétablit brutalement la circulation sanguine, on s'aperçoit que les cellules qui étaient insuffisamment irriguées meurent parce que, tout à coup, elles sont trop irriguées.

Il est nécessaire de procéder à une irrigation progressive pour assurer le succès de l'intervention. Il est donc indispensable de contrôler l'irrigation sanguine en fonction de différents paramètres qui sont, en particulier, la quantité, la température, la fréquence, la qualité d'oxygénation du sang, et la présence de drogues.

Il est connu par le document FR-A 2 502 499, de procéder à une rétroperfusion en plaçant dans le sinus coronaire une sonde à ballonnet gonflable, alimentée par une pompe. Le ballonnet sert à immobiliser la sonde dans le sinus coronaire pendant la diastole, pour autoriser la rétroperfusion de sang artériel par la pompe. Mais la rétroperfusion ne donne pas des résultats très satisfaisants.

L'un des buts de l'invention est de proposer une pompe d'assistance permettant d'amener dans la circulation coronaire du sang oxygéné contenant éventuellement les drogues.

Un autre but de l'invention est d'assurer cette assistance dans des conditions optimales d'efficacité et sans apporter de traumatisme supplémentaire.

La présente invention a pour objet une pompe de perfusion coronaire, pour assistance thérapeutique temporaire en cas d'insuffisance de la circulation coronaire, comportant une sonde munie d'un ballonnet gonflable destiné à fixer l'extrémité de la sonde dans l'artère coronaire, une pompe susceptible d'injecter par l'intermédiaire de la sonde du sang oxygéné et éventuellement des drogues, la pompe étant placée sous le contrôle d'un synchronisateur autorisant l'injection de sang essentiellement pendant la période diastolique du cycle cardiaque, caractérisée en ce que la pompe comporte un capteur de pression destiné à être placé dans l'aorte en regard des valves cardiaques, et que le synchronisateur reçoit les signaux d'un électrocardiographe et du capteur de pression et ne délivre à la pompe l'autorisation d'injecter que lorsque ces signaux sont en coïncidence, en un point défini du cycle cardiaque.

Selon d'autres caractéristiques de l'invention:

– le capteur de pression est porté par la sonde,
– la pompe reçoit le sang à injecter d'un oxygénateur,
– la pompe reçoit des drogues à injecter avec le sang, sous forme de perfusat,
– l'extrémité de la sonde comporte, au-delà du ballonnet gonflable, une série de perforations latérales disposées en spirale, afin d'éviter des problèmes d'hémolyse.

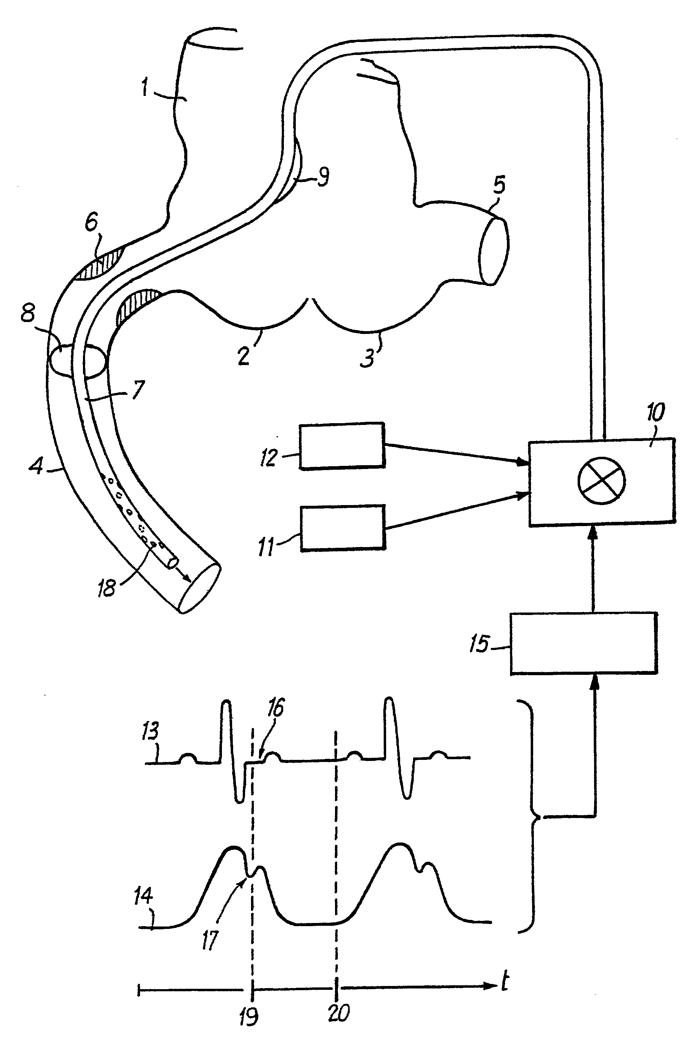

D'autres caractéristiques ressortent de la description qui suit faite avec référence au dessin annexé qui représente un schéma de principe d'une pompe de perfusion coronaire selon l'invention.

Sur le dessin, la masse cardiaque n'est pas représentée. Seules sont schématisées: l'aorte 1, les valves cardiaques correspondantes 2, 3, et les artères coronaires droite 4 et gauche 5. Dans l'exemple représenté, l'artère coronaire 4 présente un étranglement 6 entraînant une insuffisance de la circulation coronaire. Pour assurer une assistance circulatoire, l'invention prévoit de disposer une sonde 7 munie au voisinage de son extrémité d'un ballonnet gonflable 8 assurant d'une part le maintien en position de la sonde 7, dans l'artère coronaire 4, d'autre part l'occlusion étanche de l'artère coronaire 4, de façon à contrôler exactement la perfusion coronaire en aval du ballonnet. Le ballonnet 8 peut être placé soit en aval, soit en amont de l'étranglement 6. Mais l'extrémité de la sonde 7 doit s'étendre au-delà de l'étranglement. Cette extrémité de la sonde 7 est munie de perforations latérales 18, de préférence disposées en spirale, afin d'éviter les problèmes d'hémolyse. La sonde 7 porte un capteur de pression 9 qui doit être situé dans l'aorte 1, en regard des valves cardiaques 2, 3 pour fournir une indication de pression aussi bien définie que possible.

La sonde 7 est alimentée par une pompe d'assistance 10 susceptible d'injecter des quantités définies de sang oxygéné. Ce sang provient du malade lui-même par voie artérielle, ou bien d'un flacon oxygéné par un oxygénateur extérieur symbolisé en 11. Il peut recevoir des drogues, à effet local par exemple, sous forme de perfusat symbolisé en 12.

La pompe 10 doit injecter le sang, par la sonde 7, de façon pulsée, pendant la phase diastolique du cycle cardiaque, c'est-à-dire lorsque la résistance artériolaire est minimale. Il en résulte qu'à débit égal la pression nécessaire pour assurer la perfusion coronaire est minimale, ce qui permet de réduire au minimum le traumatisme résultant.

Pour définir avec précison l'instant d'injection de la pompe, on utilise à la fois les informations d'un électrocardiographe (ECG) et les pressions relevées par le capteur de pression 9, dont les tracés sont représentés respectivement en 13 (ECG) et 14 (pressions). En pratique, les signaux électriques correspondants sont adressés à un synchronisateur 15 qui ne délivre à la pompe 10 l'autorisation d'injecter du sang qu'entre les instants 19 et 20 représentés sur l'échelle des temps t. Cette autorisation n'est donnée que si les signaux reçus par le synchronisateur 15 et correspondant pour l'ECG au point 16 et pour la pression aortique au point 17 des enregistrements, sont en coïncidence.

Cette précaution est nécessaire pour s'affranchir des parasitages fréquents des ECG, et pour tenir compte des troubles éventuels du rythme cardiaque. La synchronisation des signaux de l'ECG et du capteur de pression est indispensable pour commander la perfusion coronaire, d'une part pour éviter de déclencher une perfusion à contretemps et d'autre part pour faciliter la surveillance par le personnel soignant.

La pompe d'assistance selon l'invention permet ainsi d'injecter du sang oxygéné, et éventuellement des drogues, à l'intérieur de l'artère coronaire, et pendant plusieurs heures ou plusieurs jours. Ce système d'assistance est, et doit rester, temporaire. C'est pourquoi il est prévu pour être le moins traumatique possible.

**Revendications**

1. Pompe de perfusion coronaire, pour assistance thérapeutique temporaire en cas d'insuffisance de la circulation coronaire, comportant une sonde (7) munie d'un ballonnet gonflable (8), destiné à fixer l'extrémité de la sonde dans l'artère coronaire, une pompe (10) susceptible d'injecter par l'intermédiaire de la sonde (7) du sang oxygéné et éventuellement des drogues, la pompe (10) étant placée sous le contrôle d'un synchronisateur (15) autorisant l'injection de sang essentiellement pendant la période diastolique du cycle cardiaque, caractérisée en ce que la pompe comporte un capteur de pression (9) destiné à être placé dans l'aorte en regard des valves cardiaques, et que le synchronisateur (15) reçoit les signaux d'un électrocardiographe et du capteur de pression (9) et ne délivre à la pompe (10) l'autorisation d'injecter que lorsque ces signaux sont en coïncidence, en un point défini du cycle cardiaque.

2. Pompe selon la revendication 1, caractérisée en ce que le capteur de pression (9) est porté par la sonde (7).

3. Pompe selon la revendication 1, caractérisée en ce que la pompe (10) reçoit le sang à injecter d'un oxygénateur (11).

4. Pompe selon la revendication 1, caractérisée en ce que la pompe (10) reçoit des drogues à injecter avec le sang, sous forme de perfusat (12).

5. Pompe selon la revendication 1, caractérisée en ce que l'extrémité de la sonde (7) comporte, au-delà du ballonnet gonflable (8), une série de perforations latérales (18) disposées en spirale, afin d'éviter des problèmes d'hémolyse.

**Patentansprüche**

1. Pumpe zur Perfusion in die Herzkranzgefäße zur zeitweiligen therapeutischen Assistenz im Falle einer Insuffizienz des Koronarkreislaufs, mit einer Sonde (7) mit einem aufblasbaren Luftsack (8) zum Festmachen des Sondesendes in der Koronararterie, mit einer Pumpe (10) zum Einspritzen, über die Sonde (7) von sauerstoffhaltigem Blut bzw. Arzneien wobei die Pumpe (10) von einem Synchronisationsgerät (15) gesteuert wird, das Einspritzen von Blut hauptsächlich während der diastolischen Periode des Herzzyklus erlaubt, dadurch gekennzeichnet, daß die Pumpe einen Sensor (9) zum Messen des Druckes enthält, der in die Aorta, gegenüber der Herzklappen einsetzbar ist, und daß das Synchronisationsgerät (15) Signale von Drucksensor (9) und von einem EKG-Gerät erhält, und der Pumpe die Injektionerlaubnis nur dann erteilt, wenn die Signale an einem definierten Punkt des Herzzyklus übereinstimmen.

2. Pumpe nach Anspruch 1, dadurch gekennzeichnet, daß der Druckensor (9) auf der Sonde (7) aufgesetzt ist.

3. Pumpe nach Anspruch 1 dadurch gekennzeichnet, daß die Pumpe das einzuspritzende Blut von einem Sauerstofferteiler (11) erhält.

4. Pumpe nach Anspruch 1, dadurch gekennzeichnet, daß die Pumpe die mit dem Blut einzuspritzende Arzneien in Form einer Perfusionsflüßigkeit (12) erhält.

5. Pumpe nach Anspruch 1, dadurch gekennzeichnet, daß das Ende der Sonde (7) eine Reihe von Seitenöffnungen enthält, die nach dem aufblasbaren Luftsack (8) spiralförmig angeordnet sind, um die Hemolyseprobleme zu verhindern.

**Claims**

1. Coronary perfusion pump, for temporary therapeutic assistance in case of insufficiency of the coronary circulation, including a catheter (7) provided with an inflatable balloon (8) for fixing the end of said catheter in the coronary artery, a pump (10) for injecting oxygenated blood and eventually drugs through said catheter (7), said pump (10) being placed under the control of sychronizer (15) that permits injection of blood essentially during the diastolic period of the cardiac cycle, characterized in that said pump comprises a pressure sensor (9) to be placed in the aorta opposite the cardiac valves, and said synchronizer (15) receives signals from an electrocardiogram device and from said pressure sensor (9) and sends the pump (10) the authorization to inject only when these signals coincide in one definite point of the cardiac cycle.

2. Pump according to claim 1 characterized in that said pressure sensor (9) is born by said catheter (7).

3. Pump according to claim 1 characterized in that said pump (10) receives the blood to be injected from an oxygenator (11).

4. Pump according to claim 1, characterized in that said pump (10) receives drugs to be injected with the blood in the form of a perfusate (12).

5. Pump according to claim 1 characterized in that the end of said catheter (7) comprises, beyond said inflatable balloon (8), a series of lateral perforations (18) disposed in a spiral to avoid the problems of hemolysis.